Europäisches Patentamt

European Patent Office

Office européen des brevets

(19)

(11) Veröffentlichungsnummer: **0 029 089**
**B1**

(12) **EUROPÄISCHE PATENTSCHRIFT**

(45) Veröffentlichungstag der Patentschrift:
07.09.83

(21) Anmeldenummer: 80104832.3

(22) Anmeldetag: 14.08.80

(51) Int. Cl.³: **G 01 N 33/92**

(54) Verfahren zur Diagnose von Fettstoffwechselstörungen durch quantitative Bestimmung der Oberflächenladung von Serum-Lipoproteinen.

(30) Priorität: 24.08.79 DE 2934228

(43) Veröffentlichungstag der Anmeldung:
27.05.81 Patentblatt 81/21

(45) Bekanntmachung des Hinweises auf die Patenterteilung:
07.09.83 Patentblatt 83/36

(84) Benannte Vertragsstaaten:
AT BE CH DE FR GB IT LI LU NL SE

(56) Entgegenhaltungen:
DE-A-2 600 664
DE-A-2 928 364
BIOCHEMISTRY, Band 12, Nr. 14, Juli—September 1973 Washington R. W. DeBLOIS et al. »Application of Laser Self-Beat Spectroscopic Technique to the Study of Solutions of Human Plasma Low-Density Lipoproteins« Seiten 2645 bis 2649
PROGRESS COLOID AND POLYMER SCI., Vol. 65, 1978 Darmstadt D. Horn »Optisches Zweistrahlverfahren zur Bestimmung von Polyelektrolyten in Wasser und zur Messung der Polymeradsorption an Grenzflächen« Seiten 251 bis 264

(73) Patentinhaber: BASF Aktiengesellschaft,
Carl-Bosch-Strasse 38, D-6700 Ludwigshafen (DE)

(72) Erfinder: Horn, Dieter, Dr., Schroederstrasse 69,
D-6900 Heidelberg 1 (DE)
Erfinder: Lueddecke, Erik, Dr., Altholzweg 25,
D-6700 Ludwigshafen (DE)
Erfinder: Heuck, Claus Christian, Dr., Birkenweg 20,
D-6901 Wilhelmsfeld (DE)

**0 029 089**

Verfahren zur Diagnose von Fettstoffwechselstörungen durch quantitative Bestimmung der Oberflächenladung von Serum-Lipoproteinen

Die Erfindung betrifft eine Polyelektrolyt-Titration der Serum-Lipoproteine mit einem Polykation zur Diagnose von Fettstoffwechselstörungen.

Im menschlichen Serum sind bekanntlich 3 Klassen von Lipoproteinen, die in partikulärer Form vorliegen, nachweisbar, und zwar very low density Lipoproteine (VLDL), low density Lipoproteine (LDL) und high density Lipoproteine (HDL), denen eine unterschiedliche Bedeutung für die Entwicklung der Arteriosklerose zukommt. Während die VLDL und LDL die Atherogenese fördern, wird den HDL eine protektive Wirkung zugesprochen. Zwischen den einzelnen Lipoproteinklassen besteht eine metabolische Beziehung insofern, als einzelne Bestandteile der LDL, z. B. Apolipoprotein B und Cholesterin, oder der HDL, z. B. Triglycerid oder Apolipoprotein A, aus den VLDL entstammen und bei deren biochemischen Abbau in die andere Lipoproteinklasse transferiert werden.

Weiterhin ist bekannt, daß die Zusammensetzung der einzelnen Lipoproteine von Personen mit einer primären oder auch sekundären Fettstoffwechselstörung sich von der der Lipoproteine von normolipämischen Personen deutlich unterschieden. Über die spezifischen chemischen und physiko-chemischen Oberflächeneigenschaften der einzelnen Lipoproteinklassen weiß man nur wenig. So wurde unter anderem ein erhöhter Neuraminsäuregehalt in LDL vom Typ II Hyperlipoproteinämikern festgestellt. Diese Eigentümlichkeit ist für die Verstoffwechslung der LDL bedeutsam. Ferner wurde aus elektrophoretischen Untersuchungen gefolgert, daß VLDL aus dem Serum von Personen mit einer Typ IV Hyperlipoproteinämie einen höheren isoelektrischen Punkt aufweisen als VLDL aus dem Serum von Normolipämikern.

Der Abbau oder die Ablagerung von Lipiden aus Serum-Lipoproteinen setzt eine spezifische Wechselwirkung der Lipoproteinoberfläche mit einem Enzym, einer Zellmembran oder Gebindegewebsfasern voraus. Die Bindung von Lipoprotein an eine Lipase ist für die Geschwindigkeit des enzymatischen Abbaus sogar die geschwindigkeitsbestimmende Reaktion. Phospholipide an der Substratoberfläche können den Angriff verschiedener Lipasen z. B. an einem Serumlipoprotein unterschiedlich beeinflussen.

Verschiedene Mechanismen werden diskutiert, die für die Ablagerung von Lipoproteinen in den Gefäßwänden maßgebend sind. Die Bindung von Lipoproteinen an Elastin scheint nach bisherigen Erkenntnissen über oberflächenständige Lipide zu erfolgen. Demgegenüber erfolgt die Bindung von Lipoproteinen an Zellmembranen oder Mucopolysaccharide über basische Gruppen im Proteinanteil der Lipoproteinpartikel.

Diese Beobachtungen zeigen, daß eine Änderung der Oberflächenstruktur den Metabolismus der Lipoproteine und dadurch auch die Synthese von Lipiden entscheidend beeinflussen kann. In epidemiologischer und präventiv-medizinischer Hinsicht erscheinen diese Feststellungen umso bedeutsamer, da nach bisherigen Erkenntnissen erhöhte Serumkonzentrationen von Lipiden und damit auch von bestimmten Lipoproteinen in Folge eines gestörten Fettstoffwechsels nachweisbar sind. Zahlreiche Faktoren kommen hierbei als Ursachen in Betracht. Die Bedeutung der Oberflächeneigenschaften von Lipoproteinen hat sich zwar aus zahlreichen Untersuchungen ergeben, genauere Analysen sind bisher jedoch nur in beschränktem Ausmaß erfolgt, da bisher unzureichende Methoden zur Charakterisierung der Strukturelemente der Lipoproteinoberfläche zur Verfügung stehen.

Im Bereich der Kolloidchemie sind in neueren Zeiten colorimetrische Titrationsmethoden zur Bestimmung von Polyelektrolytmolekülen entwickelt worden, z. B. Horn, D., Progress Colloid and Polymer Sci. 65, 251 bis 264 (1978). Diese Methodik ist vereinzelt auch als Trübungsmeßverfahren zur quantitativen Bestimmung von gelösten natürlichen Polymeren, z. B. Heparin, oder halbsynthetischen Polymeren, z. B. Methylglycol-Chitosan, verwendet worden. Diese quantitative Bestimmungsmethode für Polyelektrolyte kann auch verwendet werden zur Messung der Polymeradsorption an Grenzflächen disperser Teilchen. Hierzu ist es erforderlich, die mit einer Polymeradsorbatschicht belegten Teilchen vor einer Bestimmung der nicht adsorbierten im Dispergiermedium verbliebenen Polymermoleküle abzutrennen.

Es ist bekannt, daß die Lipoproteine des menschlichen Serums mit Polyanionen, wie z. B. Dextransulfat, oder auch Polykationen, wie z. B. Polyäthylenimin, Wechselwirkungen eingehen (T. Nishida und U. Cogan, J. biol. Chem. 245, 4689 bis 4697 (1970)). Diese Reaktionen sind zur Fraktionierung von Lipoprotein aus dem menschlichen Serum verwendet worden. Aus der DE-OS 2 600 664 und der Literaturstelle Heuck et al Clin. Chem. 23, 536 bis 540 (1977) geht beispielsweise hervor, daß man zur Bestimmung des $\beta$-Cholesterins in der Fraktion der LDL dem Serum Polyäthylenimin zusetzt und anschließend mit einem Kationenaustauscher VLDL und HDL extrahiert. Quantitative Aussagen über die Oberflächenladung von Lipoproteinen können mit diesen Verfahren nicht gewonnen werden.

Aufgabe der vorliegenden Erfindung ist es, ein Verfahren zur Diagnose von Fettstoffwechselstörungen durch quantitative Bestimmung der negativen Oberflächenladungen von Serum-Lipoproteinen zu entwickeln, um dadurch genauere Erkenntnisse über Faktoren, die bei der Pathogenese von Fettstoffwechselstörungen eine Rolle spielen, erhalten zu können.

2

Die Lösung der Aufgabe besteht darin, daß man zu dem vorhandenen Serum-Lipoprotein eine definierte überschüssige Menge eines Polykations in wäßriger Lösung zugibt und die nicht gebundene Menge des Polykations in Gegenwart eines metachromatischen Farbstoffs als Indikator mit einem chromotropen Polyanion zurücktitriert.

Das bei dem erfindungsgemäßen Verfahren verwendete Polykation wird an der polyanionischen Grenzfläche der vorhandenen Lipoproteine gebunden. Aus der Differenz der vorgegebenen und rücktitrierten Menge an freien Ladungen des Polykations ergibt sich die negative Oberflächenladung des Lipoproteins.

Das erfindungsgemäße Verfahren zeichnet sich durch eine überraschend einfache Meßweise und hohe Genauigkeit aus, da die Bestimmung der Oberflächenladung der zu untersuchenden Lipoproteine bis in einen Bereich von $10^{-8}$ Äquivalent/ml Lösung erfolgt. Ein weiterer Vorteil besteht darin, daß die Messung unmittelbar in der Lösung der Lipoproteinserumfraktion durchgeführt wird.

Die zu bestimmenden Lipoproteine, insbesondere die Klasse VLDL, LDL und HDL, werden in der Regel aus dem menschlichen Serum durch Ultrazentrifugation in üblicher Weise in Form einer Serumfraktion erhalten (GIT Fachz. Lab. 23, 748 bis 753 (1979)).

Die Lipoprotein enthaltende Serumfraktion wird anschließend mit einer efinierten überschüssigen Menge eines Polykations in wäßriger Lösung versetzt. Das Polykation wird an der Lipoproteinoberfläche an negativ geladenen Residuen gebunden.

Als Polykationen kommen kationische Polymere, wie Polyäthylenimin, modifizierte Polyäthylenimine oder Polydiallyldimethylammonium-Salze, insbesondere die Chloride, in Betracht.

Das besonders bevorzugte Polykation ist Polyäthylenimin. Ein erfindungsgemäß zu verwendendes Polyäthylenimin läßt sich durch ein mittels Lichtstreuung bestimmtes Gewichtsmittel des Molekulargewichts von 1000 bis 5 Millionen, entsprechend einem K-Wert von 14 bis 31, bevorzugt 20 000 bis 30 000, und einer Grenzviskosität von 7,0 bis 9,5 ml/g charakterisieren. Ein besonders geeignetes Polyäthylenimin ist unter dem Handelsnamen Polymin G500 erhältlich.

Die kationische Ladungsdichte des Polyäthylenimins liegt in Abhängigkeit des pH-Wertes zweckmäßigerweise bei einem pH 3 bei 21 meq/g, bei pH 5 bei 18 meq/g, bei pH 7 bei 15 meq/g, bei pH 9 bei 12 meq/g.

Zweckmäßigerweise werden wäßrige Standardlösungen, die 1 bis 10 mg/l, bevorzugt 2,0 bis 6 mg/l Polyäthylenimin enthalten, verwendet.

Ein besonders geeignetes Polydiallyldimethylammoniumchlorid ist erhältlich unter der Bezeichnung Cat-floc 261.

Für die Rücktitration kann grundsätzlich jedes chromotrope Polyanion verwendet werden. Zweckmäßige chromotrope Polyanionen sind die Alkalisalze, insbesondere Kalium- oder Natriumsalze, von Polyvinylsulfaten mit einem Veresterungsgrad zwischen 30 und 100%, Polyphosphate, Dextransulfate, Polyvinylalkoholsulfate mit einem Veresterungsgrad von etwa 50%, Heparin, Chondroitinsulfate, Polyacrylate und Polymethacrylate in Form ihrer wäßrigen Lösungen.

Das bevorzugte Polyanion ist Kaliumpolyvinylsulfat (PVSK).

Ein geeignetes PVSK läßt sich durch ein mittels Lichtstreuung bestimmtes Gewichtsmittel des Molekulargewichts von 1000 bis 30 000 charakterisieren und weist zweckmäßigerweise ein Veresterungsgrad von 100% und bei pH 7 eine Ladungsdichte von etwa 6 meq/g auf.

Zweckmäßigerweise werden Standardlösungen, die 100 bis 1000 mg/l, bevorzugt 300 bis 400 mg/l enthalten, verwendet.

Das überschüssige Polykation wird in Gegenwart eines gleichsinnig geladenen metachromatischen Farbstoffs als Indikator zurücktitriert. Als Indikator kommen beispielsweise in Betracht Pyronin G, Acridinorange, Methylenblau oder Toluidinblau.

Der besonders bevorzugte Indikator ist das o-Toluidinblau.

In der Regel werden die Indikatoren in Form einer wäßrigen Stammlösung, die etwa 40 mg/l enthält, verwendet. Davon werden etwa 3 ml auf 100 ml Reaktionsmedium verwendet, so daß die Indikatorkonzentration bei der Bestimmung in der Regel $10^{-7}$ bis $10^{-5}$ Mol/l beträgt.

Der Umschlagspunkt wird zweckmäßigerweise durch eine colorimetrische Meßanordnung, wie sie beispielsweise in Progress Colloid and Polymer Sci. 65. 251 bis 264 (1978) beschrieben wird, ermittelt.

Die Titration wird zweckmäßig bei Raumtemperaturen und in einem pH-Bereich zwischen 3,5 und 9, bevorzugt 6 bis 8, durchgeführt.

Hervorzuheben ist, daß niedermolekulare Ionen, anorganische Salze sowie 1- bis 3wertige Säuren und Basen oder organische Ionen, insbesondere Ammoniumsalze, unterhalb einer Grenze von etwa $10^{-2}$ Mol/l nicht störend wirken.

Aus diesem Grunde kann das erfindungsgemäße Verfahren auch in Gegenwart von Natriumchlorid oder, falls es erforderlich ist, von Pufferlösungen, beispielsweise einem Phosphatpuffer, wobei die Salzkonzentration einen Wert von $5 \times 10^{-2}$ Mol/l nicht überschreiten sollte, durchgeführt werden.

Die eigentliche Titration erfolgt zweckmäßigerweise in einer automatischen Apparatur, wie sie beispielsweise in der obengenannten Literaturstelle beschrieben wird, und in der Regel in Dosierschritten von 0,01 ml in an sich üblicher Weise bis zum Umschlagspunkt des verwendeten Indikators.

Mit Hilfe des erfindungsgemäßen Verfahrens der Bestimmung der Oberflächenladung von

Lipoproteinen wird eine differenzierte Aussage über die Oberflächeneigenschaften dieser Partikel ermöglicht. Diese Aussage erscheint im Hinblick auf den Katabolismus von Lipoproteinen und die Bindung von Lipoproteinen an Zellmembranen und Gefäßwände von besonderer Bedeutung, nachdem ein ursächlicher Zusammenhang zwischen den Oberflächeneigenschaften von Lipoproteinen des menschlichen Serums und der Ablagerung von Lipiden in den Gefäßwänden im Rahmen der Entwicklung einer Arteriosklerose nachgewiesen ist.

## Beispiel 1

1 ml einer 0,5 bis 20 mg Lipoprotein enthaltenden Fraktion wird in 100 ml einer Lösung von 0,4 mg/100 ml Polyäthylenimin (PEI) bei 23°C und bei einem pH-Wert von 7 gegeben und 15 Minuten lang gerührt.

Anschließend werden 3 ml einer Toluidinblau-Stammlösung (40 mg/l) als Indikator zugesetzt (Endkonzentration 1,2 mg/l entspricht $4 \times 10^{-6}$ Mol/l) und mit einer PVSK-Lösung einer Konzentration von 0,324 g/l ($2 \times 10^{-3}$ Mol/l) titriert.

Die Titration erfolgt in einer automatischen Apparatur wie sie in Progress Colloid and Polymer Sci. 65, 251 bis 264 (1978) beschrieben wird, in Dosierschritten von 0,01 ml bis zum Umschlagspunkt des Indikators bei einer Wellenlänge von 635 nm.

Aus der Differenz aus der eingesetzten und rücktitrierten Menge freier kationischer Zentren des PEI ergibt sich bei einer bekannten Ladungsdichte von 14,7 meq/g Polyäthylenimin und von 6,17 meq/g PVSK die zu ermittelnde negative Oberflächenladung pro Lipoproteinmenge (g).

Zur Erhöhung der Genauigkeit ist es zweckmäßig, den Verbrauch an Polyäthylenimin durch die Lipoproteine in einer Titrationsreihe mit steigender Menge eingesetzten Lipoproteins zu messen. Der Verbrauch wächst linear mit der eingesetzten Lipoproteinmenge an und aus der Geradensteigung kann die mengenbezogene Ladungsdichte ermittelt werden.

Die Umrechnung auf eine oberflächenbezogene spezifische Ladungsdichte (meq/m²) erfolgt nach Bestimmung der spezifischen Oberfläche der Lipoproteine durch Messung der Teilchengrößenverteilung der Proben nach bekannten Methoden. In Betracht kommen Ultrazentrifugation, elektronenmikroskopische Analyse oder vorteilhaft die Photonen-Korrelations-Spektroskopie. Letzteres Verfahren gestattet eine direkte Messung der translatorischen Diffusionskoeffizienten der Lipoproteine, R. W. De Blois, E. E. Uzgiris, S. K. Devi, A. M. Gotto jr., Biochemistry 12, 2645−49 (1973); und zwar werden durch Anwendung der experimentell zugänglichen Autokorrelations-Zeit-Funktion der Intensität von an den Proben gestreuten Laserlichts nach dem Verfahren von D. E. Koppel, J. Chem. Phys. 57,4814−4820 (1972) der sog. Z-Mittelwert und die Varianz des Diffusionskoeffizienten erhalten. Nach Umrechnung dieser Werte auf die dritten Momente der Radius-Verteilung erhält man daraus direkt die (volumen-)spezifische Oberfläche der Lipoprotein-Proben.

## Beispiel 2

Wie in Beispiel 1 wird 1 ml einer 0,5 bis 20 mg Lipoprotein enthaltenden Fraktion in 100 ml einer Lösung von 2,2 mg/100 ml Cat-floc 261 bei 23°C und einem pH von 7 versetzt und 15 Minuten lang gerührt.

Anschließend wird gemäß Beispiel 1 weiterverfahren.

Aus der Differenz aus der eingesetzten und rücktitrierten Menge freier kationischer Zentren des Cat-floc ergibt sich bei einer bekannten Ladungsdichte von 2,63 meq/g Cat-floc die zu ermittelnde negative Oberflächenladung pro Lipoproteinmenge (g).

In der folgenden Tabelle sind Untersuchungsergebnisse der Messung der spezifischen Oberflächenladung an Lipoproteinen, die von Personen mit verschiedenen Formen einer Hyperlipoproteinämie erhalten wurden, wiedergegeben. Die Oberflächenladung wurde, wie im Beispiel angegeben, gemessen. Die spezifische Oberfläche wurde mit Hilfe der Photonen-Korrelations-Spektroskopie-Messung berechnet.

Anhand einer Untersuchung mit isolierten Lipoproteinen von Spendern mit einer Normolipämie oder mit unterschiedlichen Formen einer Hyperlipoproteinämie (Typ IIa, IIb, III, IV, V nach Frederickson) wurde festgestellt, daß die negative Ladungsdichte der Oberfläche von LDL geringer ist als die von VLDL (Tabelle 1). Ferner ergaben die Untersuchungen, daß die negative Ladungsdichte der Oberfläche von VLDL verhältnismäßig stark variiert. Hierbei ergibt sich keine signifikante Korrelation zu der Größe der Partikel und überraschenderweise auch nicht zu deren Phospholipidgehalt. Hingegen besteht eine positive Korrelation zu dem Gehalt an Apolipoprotein B in den VLDL, für den in zahlreichen Untersuchungen eine enge Beziehung zur Entwicklung der Arteriosklerose nachgewiesen worden ist.

Der klinisch-chemische Wert der mit Hilfe des neu entwickelten Verfahrens zu erhaltenen Information erscheint um so bedeutsamer, da es durch einfache Meßweise gelingt, kausale Faktoren für den Abbau oder die Ablagerung von Lipoproteinen in den Gefäßwänden, wie z. B. die elektrostatischen Eigenschaften von Lipoproteinen für die klinische Differentialdiagnostik der primären und sekundären Fettstoffwechselstörungen zu ermitteln.

4

Tabelle 1

| Beispiel | Probe | Polykation | Polyanion | Lipo-protein (mg/ml) | Neg. Oberfl.-ladung/Vol. (meq/ml) | Lipoprotein-durchmesser ($\mu$m) | Varianz | Spez. Ober-fläche ($m^2/cm^3$) | Spez. Oberfl.-ladung (meq/ml) |
|---|---|---|---|---|---|---|---|---|---|
| 1 | LDL | PEI | PVSK | 4,6 | $1,666 \times 10^{-3}$ | 0,0300 | 0,282 | 420 | $0,862 \times 10^{-3}$ |
| 2 | LDL | PEI | PVSK | 3,39 | $1,24 \times 10^{-3}$ | 0,0306 | 0,312 | 480 | $1,762 \times 10^{-3}$ |
| 3 | LDL | Catfloc 261 | PVSK | 2,99 | $0,32 \times 10^{-3}$ | 0,0558 | 0,884 | 719 | $1,147 \times 10^{-3}$ |
| 4 | VLDL | PEI | PVSK | 2,21 | $1,47 \times 10^{-3}$ | 0,0472 | 0,298 | 281 | $2,368 \times 10^{-3}$ |
| 5 | VLDL | PEI | PVSK | 6,52 | $1,078 \times 10^{-3}$ | 0,0565 | 0,161 | 166 | $0,997 \times 10^{-3}$ |
| 6 | VLDL | PEI | PVSK | 7,15 | $1,686 \times 10^{-3}$ | 0,0829 | 0,260 | 145 | $1,629 \times 10^{-3}$ |
| 7 | VLDL | PEI | PVSK | 7,42 | $1,617 \times 10^{-3}$ | 0,0629 | 0,250 | 186 | $1,170 \times 10^{-3}$ |
| 8 | VLDL | PEI | PVSK | 3,40 | $1,029 \times 10^{-3}$ | 0,0657 | 0,339 | 218 | $1,388 \times 10^{-3}$ |
| 9 | VLDL | PEI | PVSK | 1,82 | $1,625 \times 10^{-3}$ | 0,0566 | 0,233 | 188 | $1,827 \times 10^{-3}$ |
| 10 | VLDL | PEI | PVSK | 3,15 | $1,323 \times 10^{-3}$ | 0,0602 | 0,369 | 257 | $1,632 \times 10^{-3}$ |
| 11 | VLDL | PEI | PVSK | 7,44 | $2,45 \times 10^{-3}$ | 0,0492 | 0,214 | 217 | $1,518 \times 10^{-3}$ |
| 12 | VLDL | PEI | PVSK | 5,58 | $1,47 \times 10^{-3}$ | 0,0639 | 0,137 | 138 | $1,909 \times 10^{-3}$ |
| 13 | VLDL | PEI | PVSK | 14,00 | $2,47 \times 10^{-3}$ | 0,0779 | 0,307 | 170 | $1,037 \times 10^{-3}$ |
| 14 | VLDL | Catfloc 261 | PVSK | 2,15 | $0,16 \times 10^{-3}$ | 0,0475 | 0,168 | 201 | $0,366 \times 10^{-3}$ |
| 15 | Chyclo-mikronen | PEI | PVSK | 7,68 | $0,744 \times 10^{-3}$ | 0,1257 | 0,279 | 100 | $0,968 \times 10^{-3}$ |

0 029 089

**Patentansprüche**

1. Verfahren zur Diagnose von Fettstoffwechselstörungen durch quantitative Bestimmung der Oberflächenladung von Serum-Lipoproteinen, dadurch gekennzeichnet, daß man zu dem vorhandenen Serum-Lipoprotein eine definierte überschüssige Menge eines Polykations in wäßriger Lösung zugibt und die nicht gebundene Menge des Polykations in Gegenwart eines metachromatischen Farbstoffs als Indikator mit einem chromotropen Polyanion zurücktitriert.

2. Verfahren nach Anspruch 1, dadurch gekennzeichnet, daß man als Polykation Polyäthylenimin, modifiziertes Polyäthylenimin oder ein Polydialkyldimethylammoniumsalz in wäßriger Lösung zugibt.

3. Verfahren nach Anspruch 1, dadurch gekennzeichnet, daß man als Polykation ein Polyäthylenimin mit einem K-Wert von 14 bis 31 in wäßriger Lösung zugibt.


**Claims**

1. A method of diagnosing lipometabolic disorders by the quantitative determination of the surface charge of serum lipoproteins, wherein a defined excess amount of a polycation in aqueous solution is added to the particular serum lipoprotein and the amount of polycation which has not been bonded is back-titrated against a chromotropic polyanion, in the presence of a metachromatic dye as the indicator.

2. A method as claimed in claim 1, wherein the polycation added is polyethyleneimine, modified polyethyleneimine or a polydiallyldimethylammonium salt in aqueous solution.

3. A method as claimed in claim 1, wherein the polycation added is a polyethyleneimine having a K value of from 14 to 31 in aqueous solution.


**Revendications**

1. Procédé pour le diagnostic de troubles du métabolisme des graisses par détermination quantitative de la charge superficielle de lipoprotéines du sérum, caractérisé par le fait qu'on ajoute à la lipoprotéine de sérum présente une quantité excédentaire définie d'un polycation en solution aqueuse et on titre en retour, avec un polyanion chromotrope, la quantité de polycation non liée, en présence d'un colorant métachromatique comme indicateur.

2. Procédé selon la revendication 1, caractérisé par le fait qu'on ajoute, comme polycation, de la polyéthylénimine, de la polyéthylénimine modifiée ou un sel de polydialkyldiméthylammonium, en solution aqueuse.

3. Procédé selon la revendication 1, caractérisé par le fait qu'on ajoute, comme polycation, une polyéthylénimide d'un indice K de 14 à 31, en solution aqueuse.